# EUROPÄISCHE PATENTANMELDUNG

(11) **EP 1 714 964 A1**
(43) Veröffentlichungstag der Anmeldung: **25.10.2006**
(21) Anmeldenummer: 06110843.7
(22) Anmeldetag: 08.03.2006
(51) Int. Cl.: C07D 317/34

(54) **Verfahren zur Herstellung von ungesättigten, cyclischen Orthoestern**

(30) Priorität: 22.04.2005 DE 102005018909
(71) Anmelder: DEGUSSA AG, 40474 Düsseldorf (DE)
(72) Erfinder: Ruwwe, Johannes, 53859, Niederkassel (DE); Brühl, Jutta, 46282, Dorsten (DE); Bodmann, Kerstin, 3937, Baltschieder (CH)

(57) **Zusammenfassung**

Die Erfindung betrifft ein Verfahren zur Herstellung von ungesättigten, cyclischen Orthoestern, das sich dadurch auszeichnet, dass in einer ersten Verfahrensstufe eine ungesättigte Carbonsäure mit Glycidol umgesetzt und anschließend in einer zweiten Verfahrensstufe ein Orthoester der Reaktionsmischung zugegeben wird.

## Beschreibung

Die Erfindung betrifft ein Verfahren zur Herstellung von ungesättigten, cyclischen Orthoestern.

Die ungesättigten, cyclischen Orthoester finden Verwendung in der Herstellung verschiedener Polymerblends, die Polymere aufweisen, die in der Regel als nicht miteinander mischbar gelten, wie beispielsweise Polyester und Polyolefine (EP 0 496 116 A1, EP 0 499 717 A1). Die EP 0 519 642 beschreibt die Verwendung eines Copolymers aus ungesättigten, cyclischen Orthoestern in einem Polymerblend bestehend aus Polyphenylenether und Polyester. Diese Copolymere basierend auf ungesättigten, cyclischen Orthoestern ermöglichen die Verträglichkeit zwischen diesen beiden Polymerarten.

Die Herstellung der ungesättigten, cyclischen Orthoester erfolgt gemäß dem Stand der Technik dadurch, dass zunächst Glycerin mit einem Orthoester cyclisiert wird (siehe Reaktion 1) und der erhaltene Hydroxyorthoester mit Acrylsäure- oder Methacrylsäurechlorid in Gegenwart einer Base zur Zielverbindung umgesetzt wird (siehe Reaktion 2). 4-Acryloyloxymethyl-2-methoxy-2-methyl-1,3-dioxolan ((2-Methoxy-2-methyl-1,3-dioxolan-4-yl)-methyl-acrylat, MMDA) kann beispielsweise auf diese Weise hergestellt werden, wenn der Substituent R des Säurechlorids ein Wasserstoffatom ist.

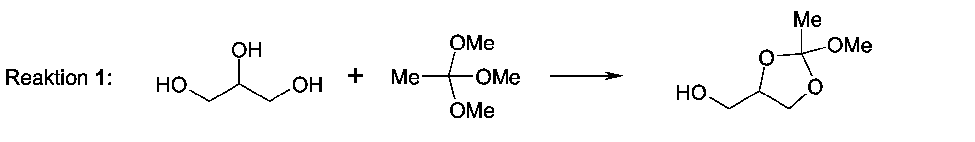

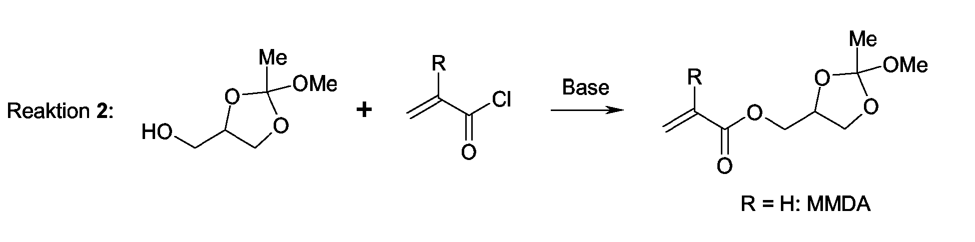

Die EP 0 471 222, EP 0 475 039 und die EP 0 475 040 beschreiben beispielsweise die Umsetzung von Glycerin (1,2,3-Propantriol) mit Methylorthoacetat in Methylenchlorid in Gegenwart von p-Toluolsulfonsäure gemäß der Reaktion 1.

Die Umsetzung des cyclisierten Orthoesters mit Acrylsäure- oder Methacrylsäurechlorid in Methylenchlorid und in Gegenwart von Triethylamin gemäß der Reaktion 2 beschreiben u.a. EP 0 496 116, EP 0 499 717, EP 0 519 642 und EP 0 731 141.

Es war die Aufgabe der vorliegenden Erfindung ein Verfahren zur Herstellung von ungesättigten, cyclischen Orthoestern zur Verfügung zu stellen, das sich durch eine gute Raum-Zeit-Ausbeute und durch den Einsatz von günstigen, kommerziell erhältlichen Rohstoffen auszeichnet.

Überraschenderweise wurde gefunden, dass durch die Umsetzung von ungesättigten Carbonsäuren, wie beispielsweise Acryl- oder Methacrylsäure, mit Glycidol und anschließender Zugabe eines Orthoesters die gewünschten ungesättigten, cyclischen Orthoester - ohne die Isolierung und Aufarbeitung des Zwischenprodukts - erhalten werden können. Das erfindungsgemäße Verfahren hat den Vorteil gegenüber Verfahren gemäß dem Stand der Technik, dass die als Edukte eingesetzte ungesättigte Carbonsäure, wie beispielsweise Acryl- oder Methacrylsäure, preisgünstiger als das entsprechende Säurechlorid, wie beispielsweise Acryl- oder Methacrylsäurechlorid, ist. Weiterer Vorteil dieses erfindungsgemäßen Verfahrens ist, dass die beiden Verfahrensstufen in ein und demselben Reaktor ohne eine Aufarbeitung der Zwischenstufen durchgeführt werden können. Die Verfahren gemäß dem Stand der Technik werden i.d.R. in Methylenchlorid durchgeführt, wobei die Reaktion 1 in hoher Verdünnung durchgeführt wird. Das erfindungsgemäße Verfahren kann dagegen ohne die Gegenwart eines Lösemittels durchgeführt werden, insbesondere kann das erfindungsgemäße Verfahren ohne die Gegenwart von halogenhaltigen Verbindungen, wie beispielsweise Methylenchlorid, durchgeführt werden.

Gegenstand der Erfindung ist ein Verfahren zur Herstellung von ungesättigten, cyclischen Orthoestern, das dadurch gekennzeichnet ist, dass in einer ersten Verfahrensstufe eine ungesättigte Carbonsäure mit Glycidol umgesetzt und anschließend in einer zweiten Verfahrensstufe ein Orthoester der Reaktionsmischung zugegeben wird.

Das erfindungsgemäße Verfahren zur Herstellung von ungesättigten, cyclischen Orthoestern, zeichnet sich dadurch aus, dass in einer ersten Verfahrensstufe eine ungesättigte Carbonsäure mit Glycidol umgesetzt und anschließend in einer zweiten Verfahrensstufe ein Orthoester der Reaktionsmischung zugegeben wird.

Unter ungesättigten, cyclischen Orthoestern werden im Rahmen dieser Erfindung Verbindungen gemäß der Struktur 1 verstanden, 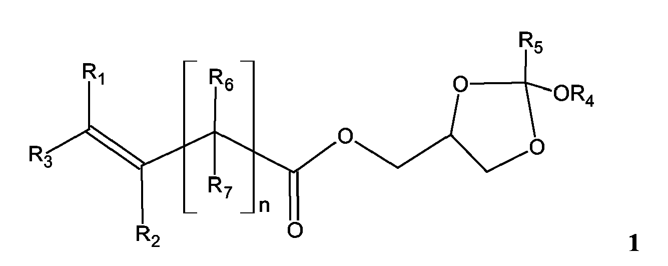 mit:
- R₁, R₂, R₃: = Wasserstoff, Alkyl- oder Arylgruppe,
- R₄: = Alkyl- oder Arylgruppe,
- R₅: = Wasserstoff, Alkyl- oder Arylgruppe,
- R₆, R₇: = Wasserstoff oder Alkylgruppe und
- n: = 0-10,
wobei die Alkyl- oder Arylgruppen jeweils substituiert oder unsubstituiert sind, die Substituenten vom Typ R₁, R₂, R₃, R₄, R₅, R₆ und/oder R₇ identisch oder verschieden sind und die Alkylgruppen verzweigt oder unverzweigt sind.

Insbesondere können gemäß dem erfindungsgemäßen Verfahren ungesättigte, cyclischen Orthoester gemäß der Struktur 1 mit n von 0 bis 4 hergestellt werden. Vorzugsweise werden gemäß dem erfindungsgemäßen Verfahren ungesättigte Verbindungen gemäß der Struktur 2 hergestellt 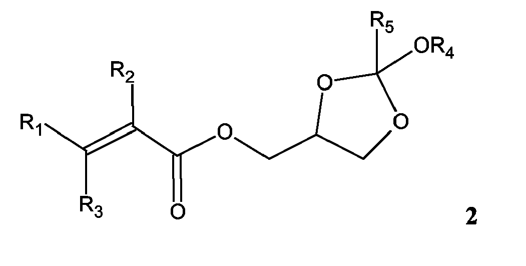 mit
- R₁, R₂, R₃: = Wasserstoff, Alkyl- oder Arylgruppe,
- R₄: = Alkyl- oder Arylgruppe und
- R₅: = Wasserstoff, Alkyl- oder Arylgruppe,
wobei die Alkyl- oder Arylgruppen jeweils substituiert oder unsubstituiert sind, die Substituenten vom Typ R₁, R₂, R₃, R₄ und/oder R₅ identisch oder verschieden sind und die Alkylgruppen verzweigt oder unverzweigt sind.

In dem erfmdungsgemäßen Verfahren wird vorzugsweise eine ungesättigte Carbonsäure der Struktur 3 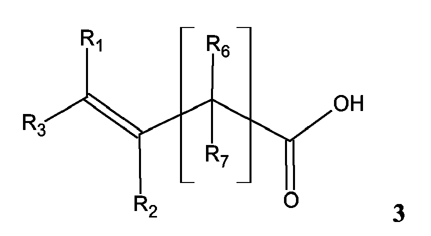 mit
- R₁, R₂, R₃: = Wasserstoff, Alkyl- oder Arylgruppe,
- R₆, R₇: = Wasserstoff oder Alkylgruppe und
- n: = 0-10,
eingesetzt, wobei die Alkyl- oder Arylgruppen jeweils substituiert oder unsubstituiert sind, die Substituenten vom Typ R₁, R₂, R₃, R₆ und/oder R₇ identisch oder verschieden sind und die Alkylgruppen verzweigt oder unverzweigt sind. Vorzugsweise werden in dem erfindungsgemäßen Verfahren ungesättigte Carbonsäuren gemäß Struktur 3 mit n von 0 bis 4 eingesetzt.

Bevorzugt werden ungesättigte Carbonsäuren gemäß Struktur 3 eingesetzt, wobei die Substituenten vom Typ R₁, R₂, R₃, R₆ und R₇ Wasserstoff oder eine Alkylgruppe mit einer Anzahl an Kohlenstoffatomen von 1 bis 6, bevorzugt jedoch Wasserstoff oder eine Methylgruppe sind. Die Alkylgruppen dieser Substituenten können hierbei sowohl verzweigt als auch unverzweigt sein, bevorzugt sind diese Substituenten jedoch unverzweigt.

Besonders bevorzugt werden als ungesättigte Carbonsäuren α,β-ungesättigte Carbonsäuren gemäß der Struktur 4 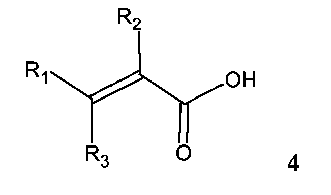 mit
- R₁, R₂ und R₃ =: Wasserstoff, Alkyl- oder Arylgruppe,
in dem erfindungsgemäßen Verfahren eingesetzt, wobei die Alkyl- oder Arylgruppen jeweils substituiert oder unsubstituiert sind, die Substituenten vom Typ R₁, R₂ und/oder R₃ identisch oder verschieden sind und die Alkylgruppen verzweigt oder unverzweigt sind.

Insbesondere sind die Substituenten vom Typ R₁ R₂ und R₃ in der Struktur 4 Wasserstoff oder eine Alkylgruppe mit einer Anzahl von Kohlenstoffatomen von 1 bis 10, bevorzugt von 1 bis 6 und besonders bevorzugt Wasserstoff oder eine Methyl- oder Ethylgruppe.

In einer besonderen Ausführungsform des erfindungsgemäßen Verfahrens ist der Substituent vom Typ R₃ der α,β-ungesättigten Carbonsäure gemäß der Struktur 4 Wasserstoff. Die Substituenten vom Typ R₁ und R₂ der α,β-ungesättigten Carbonsäure gemäß der Struktur 4 sind vorzugsweise Wasserstoff oder eine Alkylgruppe mit einer Anzahl an Kohlenstoffatomen von 1 bis 6, bevorzugt jedoch Wasserstoff oder eine Methylgruppe. In einer besonders bevorzugten Ausführungsform des erfindungsgemäßen Verfahrens werden Acrylsäure, Methacrylsäure oder Crotonsäure als α,β-ungesättigte Carbonsäure eingesetzt.

Unter Glycidol wird im Rahmen dieser Erfindung die Verbindung 2,3-Epoxy-1-propanol verstanden.

Das erfindungsgemäße Verfahren kann in einem inerten Lösemittel, wie beispielsweise Pentan, Hexan, Cyclohexan, Heptan, Methylcyclohexan, Toluol, Benzol, Chloroform, Methylenchlorid, Ether, Dimethoxyethan, Aceton, Methyl-isobutylketon, Methanol, Ethanol, Propanol, Butanol, Ethylenglycol, Glycerin, Dimethylformamid, Dimethylacetamid, durchgeführt werden.

Insbesondere kann das erfmdungsgemäße Verfahren in einem inerten und halogenfreien Lösemittel, wie beispielsweise Pentan, Hexan, Cyclohexan, Heptan, Methylcyclohexan, Toluol, Benzol, Ether, Dimethoxyethan, Aceton, Methyl-isobutylketon, Methanol, Ethanol, Propanol, Butanol, Ethylenglycol, Glycerin, Dimethylformamid, Dimethylacetamid, durchgeführt werden.

Bevorzugt wird das erfindungsgemäße Verfahren jedoch ohne den Einsatz eines Lösemittels durchgeführt. Insbesondere wird hierbei eine ungesättigte Carbonsäure eingesetzt, die bei der Reaktionstemperatur des erfmdungsgemäßen Verfahrens als Flüssigkeit vorliegt. Es wird daher vorzugsweise eine ungesättigte Carbonsäure eingesetzt, die einen Schmelzpunkt von maximal 100°C, bevorzugt von maximal 80°C und besonders bevorzugt von maximal 50°C aufweist. Das erfmdungsgemäße Verfahren kann somit sowohl in einem Einphasensystem als auch in einem Zweiphasensystem, wenn die ungesättigte Carbonsäure mit dem Glycidol bei der entsprechenden Reaktionstemperatur nicht mischbar ist, durchgeführt werden.

In einer besonderen Ausführungsform wird die ungesättigte Carbonsäure zunächst in Glycidol gelöst oder suspendiert, diese Vorgehensweise eignet sich vor allem für Carbonsäuren, die bei den Reaktionstemperaturen des erfindungsgemäßen Verfahrens nicht als Flüssigkeit vorliegen würden.

Das erfindungsgemäße Verfahren wird in der ersten Verfahrensstufe vorzugsweise bei einer Temperatur von 0°C bis 200°C, bevorzugt von 10°C bis 180°C und besonders bevorzugt von 50°C bis 150°C durchgeführt.

Die erste Verfahrensstufe des erfindungsgemäßen Verfahrens wird vorzugsweise bei einem Druck von 100 mbar bis 50 bar, bevorzugt bei einem Druck von 1 bar bis 10 bar und besonders bevorzugt bei Atmosphärendruck durchgeführt.

Als Katalysator kann in der ersten Verfahrensstufe des erfindungsgemäßen Verfahrens ein saurer Katalysator, insbesondere Brönstedt- oder Lewis-Säuren, wie beispielsweise p-Toluolsulfonsäure, HCl, H₃PO₄, HCOOH, H₂SO₄, NaHSO₄, Oxalsäure, Al(Halogen)₃, B(Halogen)₃, TiCl₄, ZnCl₂, FeCl₃, MgCl₂, eingesetzt werden. Bevorzugt wird in der ersten Verfahrensstufe p-Toluolsulfonsäure als saurer Katalysator eingesetzt.

In der ersten Verfahrensstufe des erfindungsgemäßen Verfahrens können auch basische Katalysatoren, ausgewählt aus primären, sekundären oder tertiären Aminen, eingesetzt werden, bevorzugt werden Trialkylamine und besonders bevorzugt Triethylamin als basischer Katalysator eingesetzt.

In einer besonderen Ausführungsform des erfindungsgemäßen Verfahrens wird in der ersten Verfahrensstufe ein salzartiger Katalysator, insbesondere Salze von NH₃, primären, sekundären oder tertiären Aminen, eingesetzt. Bevorzugt werden hierbei als salzartige Katalysatoren NH₄Cl, NEt₃HCl oder NBu₃HCl eingesetzt.

In einer weiteren Ausführungsform des erfindungsgemäßen Verfahrens können in der ersten Verfahrensstufe Ionentauscher, insbesondere Ionenaustauscher vom Typ Amberlyst® oder vom Typ Lewatit® eingesetzt werden. Ebenfalls können in der ersten Verfahrensstufe Katalysatoren auf der Basis von polymergebundenen Säuren, wie beispielsweise Marlon® AS3, eingesetzt werden.

In einer besonders bevorzugten Ausführungsform des erfindungsgemäßen Verfahrens wird in der ersten Verfahrensstufe ein salzartiger Katalysator, besonders bevorzugt NEt₃HCl, eingesetzt.

In der zweiten Verfahrensstufe des erfindungsgemäßen Verfahrens wird vorzugsweise ein Orthoester der Struktur 5 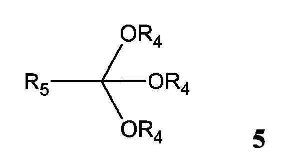 mit
- R₄: = Alkyl- oder Arylgruppe,
- R₅: = Wasserstoff, Alkyl- oder Arylgruppe,
eingesetzt, wobei die Alkyl- oder Arylgruppen jeweils substituiert oder unsubstituiert sind, die Substituenten vom Typ R₄ und R₅ identisch oder verschieden sind und die Alkylgruppen verzweigt oder unverzweigt sind. In einer besonderen Ausführungsform des erfindungsgemäßen Verfahrens werden Orthoester eingesetzt, wobei zwei der Substituenten vom Typ OR₄ durch eine Kohlenwasserstoff-Brücke, beispielsweise -O-CH₂-CH₂-O-, miteinander verbunden sind.

Bevorzugt wird in dem erfmdungsgemäßen Verfahren Trialkylorthoacetat mit Alkylgruppen, die eine Anzahl an Kohlenstoffatomen von 1 bis 6 aufweisen, als Orthoester eingesetzt. Bevorzugt werden jedoch Orthoester eingesetzt, die als Substituenten vom Typ R₄ eine Alkylgruppe mit einer Anzahl an Kohlenstoffatomen von 1 bis 4 aufweisen, insbesondere solche die als Substituenten vom Typ R₄ eine Methyl-, Ethyl-, n-Propyl-, iso-Propyl-, n-Butyl, iso-Butyl- oder sek.-Butylgruppe aufweisen. Besonders bevorzugt wird als Orthoester Trimethylorthoacetat eingesetzt.

Das erfmdungsgemäße Verfahren wird in der zweiten Verfahrensstufe vorzugsweise bei einer Temperatur von 0°C bis 200°C, bevorzugt von 10°C bis 100°C und besonders bevorzugt von 15°C bis 90°C durchgeführt. In einer bevorzugten Ausführungsform des erfindungsgemäßen Verfahrens wird die zweite Verfahrensstufe bei einer niedrigeren Temperatur durchgeführt als die erste Verfahrensstufe.

Die zweite Verfahrensstufe des erfindungsgemäßen Verfahrens wird vorzugsweise bei einem Druck von 100 mbar bis 50 bar, bevorzugt bei einem Druck von 1 bar bis 10 bar und besonders bevorzugt bei Atmosphärendruck durchgeführt.

Als Katalysator kann in der zweiten Verfahrenstufe des erfmdungsgemäßen Verfahrens ein saurer oder ein salzartiger Katalysator eingesetzt werden.

Als Katalysator kann in der zweiten Verfahrensstufe des erfindungsgemäßen Verfahrens ein saurer Katalysator, insbesondere Brönstedt- oder Lewis-Säuren, wie beispielsweise p-Toluolsulfonsäure, HCl, H₃PO₄, HCOOH, H₂SO₄, NaHSO₄, Oxalsäure, Al(Halogen)₃, B(Halogen)₃, TiCl₄, ZnCl₂, FeCl₃, MgCl₂, eingesetzt werden. Bevorzugt wird in der zweiten Verfahrensstufe p-Toluolsulfonsäure als saurer Katalysator eingesetzt.

In einer besonderen Ausführungsform des erfindungsgemäßen Verfahrens wird in der zweiten Verfahrensstufe ein salzartiger Katalysator, insbesondere Salze von NH₃, primären, sekundären oder tertiären Aminen, eingesetzt. Bevorzugt werden hierbei als salzartige Katalysatoren NH₄Cl, NEt₃HCl oder NBu₃HCl eingesetzt.

In einer weiteren Ausführungsform des erfindungsgemäßen Verfahrens können in der zweiten Verfahrensstufe Ionentauscher, insbesondere Ionenaustauscher vom Typ Amberlyst® oder vom Typ Lewatit® eingesetzt werden. Ebenfalls können in der zweiten Verfahrensstufe Katalysatoren auf der Basis von polymergebundenen Säuren, wie beispielsweise Marlon® AS3, eingesetzt werden.

In einer besonderen Ausführungsform des erfindungsgemäßen Verfahrens werden für die beiden Verfahrensstufen verschiedene Katalysatoren eingesetzt, besonders bevorzugt wird hierbei in der ersten Verfahrensstufe ein basischer Katalysator, insbesondere Triethylamin, und in der zweiten Verfahrensstufe ein saurer Katalysator, insbesondere p-Toluolsulfonsäure, eingesetzt. In dem erfmdungsgemäßen Verfahren kann jedoch auch ein und derselbe Katalysator für beide Verfahrensstufen eingesetzt werden, so dass auf eine zusätzliche Katalysatorzugabe in der zweiten Verfahrensstufe verzichtet werden kann. Bevorzugt werden hierfür ein saurer oder ein salzartiger Katalysator für beide Verfahrensstufen eingesetzt.

Die beiden Verfahrensstufen des erfindungsgemäßen Verfahrens können in demselben Reaktor durchgeführt werden, ohne dass eine Aufarbeitung von Zwischenstufen notwendig ist.

Sowohl die erste als auch zweite Verfahrensstufe des erfindungsgemäßen Verfahrens kann kontinuierlich oder diskontinuierlich durchgeführt werden.

Vorzugsweise wird in dem erfindungsgemäßen Verfahren die ungesättigte Carbonsäure vorgelegt und die weiteren Edukte, wie beispielsweise das Glycidol in der ersten Verfahrensstufe oder der Orthoester gemäß der Struktur 3 in der zweiten Verfahrensstufe werden sukzessive zudosiert, insbesondere zugetropft. Das Zutropfen der Edukte kann über mehrere Stunden erfolgen.

Dient das Glycidol als Lösemittel oder Suspensionsmittel für die ungesättigte Carbonsäure, so kann das Zutropfen des Glycidols in der ersten Verfahrensstufe teilweise oder vollständig entfallen.

Das Reaktionsgemisch kann nach der zweiten Verfahrensstufe mittels einer Destillation von den in dem Reaktionsgemisch enthaltenen Leichtsiedem befreit werden. Anschließend kann das Produkt mittels einer Vakuumdestillation gereinigt werden.

Die nachfolgenden Beispiele sollen das erfindungsgemäße Verfahren zur Herstellung von ungesättigten, cyclischen Orthoestern näher erläutern, ohne dass die Erfindung auf diese Ausführungsform beschränkt sein soll.

### Beispiel 1

115 g Acrylsäure und 7,7 g Triethylamin-Hydrochlorid werden zusammen mit 0,7 g Hydrochinon in einem Rundkolben vorgelegt und auf 85°C erhitzt. Zu dieser Eduktmischung werden 120 g Glycidol zugetropft und die erhaltene Lösung wird für 0,5 Stunden bei 85°C gerührt. Anschließend wird die Reaktionsmischung auf 75°C abgekühlt. Zu dieser Reaktionsmischung werden nun 85 g Trimethylorthoacetat zugetropft, anschließend wird für 1 Stunde bei 70°C gerührt. Das Reaktionsgemisch wird danach mittels einer Destillation von den enthaltenen Leichtsiedem befreit. Anschließend wird eine Vakuumdestillation bei 4 mbar und 95°C durchgeführt. Man erhält 4-Acryloyloxymethyl-2-methoxy-2-methyl-1,3-dioxolan in einer Ausbeute von 85%.

### Beispiel 2

47 g Acrylsäure und 3,04 g p-Toluolsulfonsäure werden zusammen mit 0,2 g Hydrochinon in einem Rundkolben vorgelegt und auf 104°C erhitzt. Zu dieser Eduktmischung werden 40 g Glycidol zugetropft und die erhaltene Lösung wird für 45 Minuten bei 100°C gerührt. Anschließend wird die Reaktionsmischung auf 25°C abgekühlt. Zu dieser Reaktionsmischung werden 84 g Trimethylorthoacetat zugetropft, anschließend wird für eine Stunde bei 25°C gerührt. Das Reaktionsgemisch wird danach mittels einer Destillation von den enthaltenen Leichtsiedem befreit. Anschließend wird eine Vakuumdestillation bei 4 mbar und 95°C durchgeführt. Man erhält 4-Acryloyloxymethyl-2-methoxy-2-methyl-1,3-dioxolan in einer Ausbeute von 56 %.

### Beispiel 3

47 g Acrylsäure und 1,62 g Triethylamin werden zusammen mit 0,2 g Hydrochinon in einem Rundkolben vorgelegt und auf 70°C erhitzt. Zu dieser Eduktmischung werden 40 g Glycidol zugetropft und die erhaltene Lösung wird für 45 Minuten bei 70°C gerührt. Anschließend wird die Reaktionsmischung auf 25°C abgekühlt. Zu dieser Reaktionsmischung werden 3 g p-Toluolsulfonsäure zugegeben, anschließend werden 84 g Trimethylorthoacetat zugetropft und anschließend für eine Stunde bei 25°C gerührt. Das Reaktionsgemisch wird danach mittels einer Destillation von den enthaltenen Leichtsiedem befreit. Anschließend wird eine Vakuumdestillation 4 mbar und 95°C durchgeführt. Man erhält 4-Acryloyloxymethyl-2-methoxy-2-methyl-1,3-dioxolan in einer Ausbeute von 45 %.

### Beispiel 4

Glycidol und Acrylsäure, die 6,4 Gew.-% Triethylamin-Hydrochlorid und 0,6 Gew.-% Hydrochinon, enthält, werden gemeinsam in einen auf 125°C erwärmten Schlaufenreaktor dosiert. Die Dosierrate für Glycidol beträgt 40 g/h, die für die Acrylsäure/Hydrochinon/Katalysatorlösung beträgt 41 g/h. Die Verweilzeit beträgt ca. 15 min. Der Ablauf des Schlaufenreaktors innerhalb einer Stunde wird aufgefangen, abgekühlt und mit 69 g Trimethylorthoacetat versetzt. Die Mischung wird 1 Stunde bei 25°C gerührt. Man erhält ein Rohprodukt des 4-Acryloyloxymethyl-2-methoxy-2-methyl-1,3-dioxolans in einer Ausbeute von 63 % per GC-Analyse.

## Patentansprüche

1. Verfahren zur Herstellung von ungesättigten, cyclischen Orthoestern,
**dadurch gekennzeichnet,**
**dass** in einer ersten Verfahrensstufe eine ungesättigte Carbonsäure mit Glycidol umgesetzt und anschließend in einer zweiten Verfahrensstufe ein Orthoester der Reaktionsmischung zugegeben wird.

2. Verfahren gemäß Anspruch 1,
**dadurch gekennzeichnet,**
**dass** eine ungesättigte Carbonsäure gemäß der Struktur 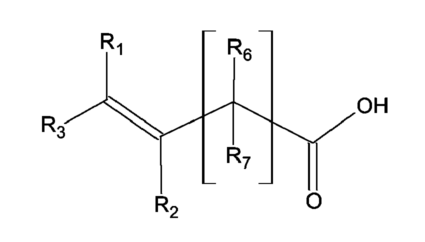 mit:
R₁, R₂, R₃ = Wasserstoff, Alkyl- oder Arylgruppe,
R₆, R₇ = Wasserstoff oder Alkylgruppe und
n = 0-10,
eingesetzt wird, wobei die Alkyl- oder Arylgruppen jeweils substituiert oder unsubstituiert sind, die Substituenten vom Typ R₁, R₂, R₃, R₆ und/oder R₇ identisch oder verschieden sind und die Alkylgruppen verzweigt oder unverzweigt sind.

3. Verfahren gemäß Anspruch 2,
**dadurch gekennzeichnet,**
**dass** als ungesättigte Carbonsäure eine α,β-ungesättigte Carbonsäure gemäß der Struktur 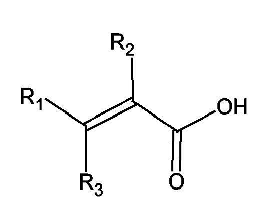 mit
R₁, R₂ und R₃ = Wasserstoff, Alkyl- oder Arylgruppe,
eingesetzt wird, wobei die Alkyl- oder Arylgruppen jeweils substituiert oder unsubstituiert sind, die Substituenten vom Typ R₁, R₂ und/oder R₃ identisch oder verschieden sind und die Alkylgruppen verzweigt oder unverzweigt sind.

4. Verfahren gemäß Anspruch 3,
**dadurch gekennzeichnet,**
**dass** als α,β-ungesättigte Carbonsäure Acrylsäure, Methacrylsäure oder Crotonsäure eingesetzt wird.

5. Verfahren gemäß zumindest einem der Ansprüche 1 bis 4,
**dadurch gekennzeichnet,**
**dass** die erste Verfahrensstufe bei einer Temperatur von 50°C bis 150°C durchgeführt wird.

6. Verfahren gemäß zumindest einem der Ansprüche 1 bis 5,
**dadurch gekennzeichnet,**
**dass** ein und derselbe Katalysator für beide Verfahrensstufen eingesetzt wird.

7. Verfahren gemäß Anspruch 6,
**dadurch gekennzeichnet,**
**dass** ein saurer Katalysator eingesetzt wird.

8. Verfahren gemäß Anspruch 6,
**dadurch gekennzeichnet,**
**dass** ein salzartiger Katalysator eingesetzt wird.

9. Verfahren gemäß zumindest einem der Ansprüche 1 bis 5,
**dadurch gekennzeichnet,**
**dass** für die beiden Verfahrensstufe verschiedene Katalysatoren eingesetzt werden.

10. Verfahren gemäß Anspruch 9,
**dadurch gekennzeichnet,**
**dass** in der ersten Verfahrensstufe ein basischer Katalysator und in der zweiten Verfahrensstufe ein saurer Katalysator eingesetzt wird.

11. Verfahren gemäß zumindest einem der Ansprüche 1 bis 10,
**dadurch gekennzeichnet,**
**dass** in der zweiten Verfahrensstufe ein Orthoester der Struktur 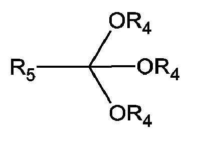 mit:
R₄ = Alkyl- oder Arylgruppe,
R₅ = Wasserstoff, Alkyl- oder Arylgruppe,
eingesetzt wird, wobei die Alkyl- oder Arylgruppen jeweils substituiert oder unsubstituiert sind, die Substituenten vom Typ R₄ und R₅ identisch oder verschieden sind und die Alkylgruppen verzweigt oder unverzweigt sind.

12. Verfahren gemäß zumindest einem der Ansprüche 1 bis 11,
**dadurch gekennzeichnet,**
**dass** beide Verfahrensstufen in demselben Reaktor durchgeführt werden.
